# EUROPÄISCHE PATENTSCHRIFT

(11) **EP 4 012 460 B1**
(45) Veröffentlichungstag und Bekanntmachung des Hinweises auf die Patenterteilung: **19.11.2025**
(21) Anmeldenummer: 20212462.4
(22) Anmeldetag: 08.12.2020
(51) Int. Cl.: G01T 1/24

(54) **VERFAHREN ZUM BETRIEB EINES STRAHLUNGSDETEKTORS SOWIE STRAHLUNGSDETEKTOR**
RADIATION DETECTOR AND METHOD FOR OPERATING A RADIATION DETECTOR
PROCÉDÉ DE FONCTIONNEMENT D'UN DÉTECTEUR DE RAYONNEMENT AINSI QUE DÉTECTEUR DE RAYONNEMENT

(43) Veröffentlichungstag der Anmeldung: 15.06.2022
(73) Patentinhaber: Siemens Healthineers AG, 91301 Forchheim (DE)
(72) Erfinder: Eismann, Alfons, 91361 Pinzberg (DE); Sturm, Leander, 95490 Mistelgau (DE)
(74) Vertreter: Siemens Healthineers Patent Attorneys

(56) Entgegenhaltungen:
- WO-A2-2014/132232
- DE-A1- 102015 201 494
- DEDIC V ET AL: "De-polarization of a CdZnTe radiation detector by pulsed infrared light", APPLIED PHYSICS LETTERS, A I P PUBLISHING LLC, US, vol. 107, no. 3, 20 July 2015 (2015-07-20), XP012199066, ISSN: 0003-6951, [retrieved on 19010101], DOI: 10.1063/1.4927382

## Beschreibung

Die Erfindung betrifft ein Verfahren zum Betrieb eines Strahlungsdetektors mit den Merkmalen des Oberbegriffs des Anspruchs 1. Weiterhin betrifft die Erfindung einen Strahlungsdetektor mit den Merkmalen des Oberbegriffs des Anspruchs 9.

Bildgebende Vorrichtungen in der medizinischen Diagnostik, beispielsweise Röntgenvorrichtungen weisen typischerweise einen Strahlungsdetektor, insbesondere Röntgenstrahlungsdetektoren, kurz: Röntgendetektoren, auf.

Röntgendetektoren sind üblicherweise als Szintillatordetektoren oder als Detektoren mit Direktkonverter ausgebildet.

Szintillatordetektoren weisen ein Szintillatormaterial auf. Szintillatormaterialien werden durch eine Bestrahlung mit Röntgenstrahlung angeregt und emittieren die Anregungsenergie in Form von Licht. Das emittierte Licht wird anschließend beispielsweise mittels Photodioden in ein elektrisches Signal umgewandelt und in einer Auswerteeinheit ausgewertet. Häufig weisen Szintillatordetektoren mehrere Szintillatorelemente auf, welche nach Art eines Arrays angeordnet sind. Analog weisen die Photodioden ebenfalls eine Anordnung nach Art eines Arrays auf.

Detektoren mit Direktkonvertern weisen üblicherweise ein Halbleitermaterial, beispielsweise einen Halbleiter auf Cadmium-Tellur-Basis auf, welcher auf ihn auftreffende Strahlung, beispielsweise Röntgenstrahlung in ein elektrisches Signal umwandelt.

Die Funktionsweise eines Direktkonverter-Detektors lässt sich grob vereinfacht wie folgt beschreiben: Die auf den Halbleiter auftreffende Röntgenstrahlung erzeugt innerhalb des Halbleiters Ladungsträger in Form von Elektronen-Loch-Paaren. Am Halbleiter ist im Betrieb eine Spannung angelegt, die sogenannte Biasspannung. Diese erzeugt ein elektrisches Feld (E-Feld) in dem Halbleiter. Die Elektronen-Loch-Paare erfahren aufgrund des E-Feldes eine Kraft, werden voneinander getrennt und bewegen sich in Richtung zu elektrischen Kontaktelementen, welche zum Anlegen der Biasspannung jeweils an einer Oberseite des Halbleiterelements und an einer Unterseite des Halbleiters angeordnet sind. Ein hierdurch entstandener Ladungspuls wird anschließend mittels einer Aufnahmeeinheit erfasst und ausgewertet. Eine genaue Beschreibung der Funktionsweise eines Direktkonverter-Detektors ist beispielsweise aus der DE 2015 201 494 A1 zu entnehmen.

Bei Bestrahlung durch Röntgenstrahlung, insbesondere bei einer hohen Intensität der Röntgenstrahlung zeigen direktkonvertierende Detektoren häufig eine Polarisation. Die Polarisation beschreibt eine unerwünschte Veränderung des E-Feldes innerhalb des Halbleitermaterials des Detektors. Die Polarisation wirkt sich somit unmittelbar auf die Detektoreigenschaften, beispielsweise die Ladungsträgertransporteigenschaft des Halbleitermaterials aus.

Die Polarisation ist häufig eine Folge von Defektstellen, beispielsweise in Form von Vakanzen, welche bei der Fertigung des Halbleiters auftreten. Zudem weist die Polarisation eine Abhängigkeit im Hinblick auf die Temperatur des Halbleitermaterials und / oder der einwirkenden Strahlung auf. Mit anderen Worten: Die Polarisationseffekte eines direktkonvertierenden Detektors sind zum einen produktionsbedingter und zum anderen betriebsbedingter Natur. Letzteres führt zu einer betriebsbedingten und somit zeitbedingten Abhängigkeit der Polarisationseffekte. D.h. der Polarisationszustand eines Detektors ist zeitlich veränderlich, beispielsweise weist ein Detektor vor einer Behandlung einen anderen Polarisationszustand auf als nach einer Behandlung.

Insbesondere ändert sich die Signalcharakteristik eines empfangenen Messsignals durch die Polarisation. Beispielsweise ändert sich die Intensität des Messsignals mit der Zeit bei gleichbleibender Strahlungsintensität beziehungsweise Strahlungsdosis. Dieser Effekt wird auch als Signaldrift bezeichnet und wirkt sich negativ auf die Funktionsfähigkeit des Detektors aus.

In der DE 10 2015 201 494 A1 ist ein Verfahren zur Ermittlung eines Polarisationszustandes eines Röntgendetektors, im Folgenden als Detektor bezeichnet, angegeben um die Signaldrift zu reduzieren.

Hierbei wird der Detektor mit einer Lichtpulsfolge beleuchtet. Die einzelnen Lichtpulse wiesen dabei unterschiedliche Intensitäten auf. Weiterhin wird eine Intensität der Lichtpulse ermittelt, bei der ein vom Detektor generierter Ladungspuls eine voreingestellte Schwellenspannung überschreitet. Die Schwellenspannung wird beispielsweise mittels einer Signalerfassungseinheit eingestellt. Mit anderen Worten: Es wird eine Beziehung zwischen einer Beleuchtungsintensität und der ermittelten überschrittenen Schwellenspannung hergestellt. Diese Beziehung variiert je nach Polarisationszustand des Detektors, wodurch eine Bestimmung des Polarisationszustandes des Detektors erfolgen kann. Infolgedessen kann der Detektor durch geeignete Maßnahmen wie beispielsweise eine Kalibrierung auf die Signaldrift eingestellt werden, d.h. der Detektor wird derart eingestellt, dass die Verschiebung des Messsignals reduziert und / oder ausgeglichen wird.

Für eine Ansteuerung der LEDs ergeben sich bestimmte Anforderungen, beispielsweise im Hinblick auf Schaltzeiten der für die Ansteuerung eingesetzten Bauteile. Um diesen Anforderungen zu genügen, werden heutzutage komplexe und teure Bauteile eingesetzt.

Ausgehend hiervon liegt der Erfindung die Aufgabe zugrunde, mit einem einfachen Aufbau einen zuverlässigen Betrieb eines Strahlungsdetektors zu ermöglichen

Die Aufgabe wird erfindungsgemäß gelöst durch ein Verfahren zum Betrieb eines Strahlungsdetektors mit den Merkmalen des Anspruchs **1.**

Vorteilhafte Ausgestaltungen, Varianten und Weiterbildungen sind Gegenstand der Unteransprüche.

Der Strahlungsdetektor wird im Betrieb mittels einer Beleuchtungseinheit, insbesondere einer LED-Einheit, zur Ermittlung einer Polarisation mit Licht beleuchtet. Dies dient zur definierten Einstellung des Strahlungsdetektors, wie es beispielsweise aus der DE 10 2015 201 494 A1 bekannt ist.

Bei dem Strahlungsdetektor handelt es sich um einen direktkonvertierenden Detektor, wie er eingangs beschrieben wurde.

Die Beleuchtungseinheit weist eine Ansteuervorrichtung auf. Die LEDs werden durch ein Ansteuersignal angesteuert, welches mit Hilfe der Ansteuervorrichtung erzeugt wird. Das Ansteuersignal wird zweckdienlicherweise aus einer Überlagerung von zwei Signalteilen gebildet. D.h., dass das Signal einen gepulsten Signalteil und einen über den gepulsten Signalteil gelagerten Gleichanteil aufweist. In einer Ausführungsform, die nicht unter die beanspruchte Erfindung fällt, können die LEDs im Betrieb ebenso lediglich mit dem gepulsten Signalteil angesteuert werden. Dies resultiert aufgrund dessen, dass die Ansteuervorrichtung eine Gleichstromquelle (DC-Stromquelle) und eine Pulsstromquelle aufweist.

Sinngemäß wird der gepulste Signalanteil von der Pulsstromquelle erzeugt und der Gleichanteil von der DC-Stromquelle erzeugt. Somit werden die beiden Signalteile zunächst getrennt voneinander erzeugt. Anschließend werden der gepulste Signalanteil und der Gleichanteil überlagert und den LEDs zugeführt. Sinngemäß handelt es sich bei dem Ansteuersignal um einen elektrischen Strom, welcher durch die LEDs geleitet wird. Der elektrische Strom, im Folgenden als LED-Strom bezeichnet, steht in unmittelbarer Korrelation mit der Intensität der LEDs. Steigt beispielsweise der Wert des LED-Stroms, so steigt auch der Wert der Intensität der LEDs.

Zur Überlagerung von gepulstem Signalteil und Gleichanteil wird der Wert des Gleichanteils auf alle Werte des gepulsten Signals addiert. Mit anderen Worten: In einem kartesischen Koordinatensystem betrachtet "verschiebt" der Gleichanteil den gepulsten Signalanteil um den Wert des Gleichanteils entlang der Ordinaten-Achse.

Aufgrund der Beaufschlagung der LEDs und somit des Halbleitermaterials des Strahlungsdetektors mittels des Gleichanteils des Ansteuersignals wird eine Kurzzeit-Driftreduktion der Elektronen-Loch-Paare erreicht. D.h. die Signalstabilität bleibt über die Zeit konstant, da der Strahlungsdetektor eine geringere Signaldrift aufweist als ohne eine Beaufschlagung des Halbleitermaterials mit dem Gleichanteil des Ansteuersignals. Der Vorteil ist, dass die Bildqualität gesteigert wird.

Mittels des gepulsten Signalteils wird bei einer ersten Messung innerhalb des Halbleitermaterials des Strahlungsdetektors ein kurzzeitiger Elektronen-Transport der Elektronen-Loch-Paare erzeugt. Die Elektronen-Transporte generieren innerhalb des Halbleitermaterials die eingangs bereits beschriebenen Ladungspulse. Die Ladungspulse generieren innerhalb einer aus der DE 10 2015 201 494 A1 bekannten Signalerfassungseinheit, beispielsweise einer Anwender-Spezifischen-Integrierten-Schaltung (ASIC) Zählereignisse, welche mittels des ASICs erfasst und gespeichert werden. Nach einer definierten Betriebsstundenzahl, vorzugsweise innerhalb eines Tages, beispielsweise nach 3 Stunden wird der Strahlungsdetektor bei einer zweiten Messung erneut mit dem gepulsten Signalteil beaufschlagt und die Ladungspulse erfasst sowie das Zählereignisse erfasst und gespeichert. Die bei der zweiten Messung erfassten und gespeicherten Zählereignisse werden anschließend mit den Zählereignissen der ersten Messung verglichen. Eine Abweichung der beiden Zählereignisse wird dann als ein Korrekturwert für gemessene Signale im Betrieb des Strahlungsdetektors mittels Röntgenstrahlung herangezogen. Der Vorteil hierbei ist, dass die Signaldrift des Strahlungsdetektors, insbesondere die Langzeitdrift verbessert und / oder verhindert wird.

Der gepulste Signalteil weist zu seiner Amplitude auch ein insbesondere periodisches Pulsmuster, bevorzugt ein Rechtecksignal auf, welche beide mittels der Pulsstromquelle eingestellt werden. Unter periodischem Pulsmuster wird hierbei verstanden, dass sich ein Signalimpuls, bevorzugt ein Rechteckimpuls, in gleichbleibenden, wiederkehrenden Zeitintervallen wiederholt.

Hierzu weist die Pulsstromquelle gemäß einer bevorzugten Ausgestaltung ein erstes Schaltelement, vorzugsweise eine elektronische Last, zum Einstellen der Amplitude und somit der Pulshöhe des gepulsten Signals auf. Weiterhin weist die Pulsstromquelle ein sich an das erste Schaltelement seriell anschließendes zweites Schaltelement, vorzugsweise einen Schalter auf, mittels welchem das Pulsmuster des gepulsten Signalteils eingestellt wird.

Dieser Ausgestaltung liegt die Überlegung zugrunde die Erzeugung des gepulsten Signalteils auf zwei Schaltelemente "zu verteilen" um eine einfache und kostengünstige Erzeugung des gepulsten Signalteils zu ermöglichen. Zur Einstellung der Pulshöhe des gepulsten Signalteils weist das erste Schaltelement handelsübliche Schalter, vorzugsweise Transistoren auf. Das zweite Schaltelement weist ebenfalls handelsübliche Schalter, insbesondere Transistoren auf. Der Unterschied der Schalter des ersten Schaltelementes und der Schalter des zweiten Schaltelementes ist in der Schaltzeit der Schalter zu sehen. Mit anderen Worten: Die Schalter des ersten Schaltelements haben, schaltungstechnisch betrachtet, eine langsamere Schaltzeit als die Schalter des zweiten Schaltelements über die das Pulsmuster des gepulsten Signalteils eingestellt wird. Somit ist für die Erzeugung des gepulsten Signalteils lediglich ein "schnell schaltendes" Bauteil erforderlich. Zur Erzeugung des gepulsten Signalteils wird zuerst ein Strom über das erste Schaltelement mit einer Amplitude eingestellt, welcher anschließend durch das zweite Schaltelement fließt und dort beispielsweise durch ein wiederholtes Ab- und wieder Zuschalten des zweiten Schaltelementes zu einem Rechtecksignal mit einer definierten Flankensteilheit und einer definierten Pulsbreite ausgebildet zu werden. Unter Flankensteilheit wird hierbei eine Zeitspanne verstanden, die das Ansteuersignal benötigt, um auf den vollen Wert der Amplitude zu erreichen. Weist beispielsweise das Ansteuersignal eine Flankensteilheit von 10ns und eine Amplitude von 100mA auf, so steigt der Wert des Ansteuersignals nach einem Einschalten innerhalb von 10ns auf den Wert von 100mA.

Unter Pulsbreite wird vorliegend die Zeitspanne verstanden, in der, beispielsweise ein Rechtecksignal die volle Amplitude aufweist bevor das Signal wieder abfällt. Weist ein Rechtecksignal beispielsweise eine Pulsbreite von 200ns und eine Amplitude von 150mA auf, so bleibt der Wert des Rechtecksignals nach einem Ansteigen auf die Amplitude von 150mA über 200ns lang konstant bevor er beispielsweise wieder auf den Wert von 0A abfällt. Die Pulsbreite bildet somit die "Breite" des Rechteckes des Rechtecksignals.

Bevorzugt weist der gepulste Signalteil der Ansteuervorrichtung eine Folge von Einzelpulsen auf, deren Amplitude zunimmt. Mit anderen Worten: Als gepulster Signalteil wird ein Signal erzeugt, welches eine Folge von Einzelpulsen aufweist. Jeder Einzelpuls weist eine Amplitude (Pulshöhe) auf. Vorzugsweise wird der gepulste Signalteil derart eingestellt, sodass die Amplitude der Einzelpulse über ein Zeitintervall, beispielsweise im Bereich von 1µs bis 5µs, konstant gehalten wird. Anschließend weist die darauf folgende Pulsfolge eine höhere Amplitude auf. Durch diese Ausgestaltung erfolgt die Zunahme der Amplitude der Einzelpulse nach Art einer Treppenform. Alternativ weist jeder Einzelpuls eine höhere Amplitude als der vorherige Einzelpuls auf, was zu einer rampenförmigen Zunahme der Amplitude der Einzelpulse führt.

Vorzugsweise werden die Ladungspulse, wie eingangs beschrieben, schaltungstechnisch so lange in ihrem Wert gesteigert, bis diese einen Wert einer voreingestellten Schwellenspannung einer Signalerfassungseinheit überschreiten. Unter Überschreiten ist vorliegend zu verstehen, dass der Wert der Ladungspulse zumindest den Wert der voreingestellten Schwellenspannung aufweist, vorzugsweise einen größeren Wert als den Wert der Schwellenspannung aufweist.

Gemäß einer besonders bevorzugten Ausgestaltung wird auf eine Regelung einer absoluten Amplitudengenauigkeit des gepulsten Signalteils verzichtet. Dieser Ausgestaltung liegt folgender Gedanke zugrunde: Um einer Kalibrierung und somit einer Reduzierung der Signaldrift Rechnung zu tragen, ist, wie bereits beschrieben, ein Vergleich der durch zumindest zwei Messungen ermittelten Zählereignissen relevant. Hierbei ist eine Erfassung der relativen Amplitudenänderung des gepulsten Signalteils, insbesondere hinsichtlich des Ampere-Wertes, hinsichtlich einer Kalibrierung des Strahlungsdetektors einer Erfassung der absoluten Amplitudenänderung vorzuziehen.

Mit anderen Worten: Weist beispielsweise ein Unterschied der Zählereignisse hinsichtlich der Amplitude des Ansteuersignals einen Wert von 20mA auf, so sind die 20mA als relativer Unterschied zwischen den beiden Messungen für eine Kalibrierung notwendig. Ob der ermittelte Unterschied von 20mA aufgrund eines Amplitudenunterschiedes von 100mA auf 120mA oder aufgrund eines Amplitudenunterschiedes von 60mA auf 80mA eintritt, ist hinsichtlich des Verfahrens zur Reduzierung der Signaldrift ein nachgeordneter Aspekt.

Zweckmäßigerweise weist die Pulsfolge jedoch bei den zumindest zwei Messungen eine gleiche Rampenform sowie einen gleichen Startwert auf. Die Amplitude der Rampenform liegt vorzugsweise bei einem Wert im Bereich von einigen hundert Milliampere.

Zweckdienlicherweise weist die DC-Stromquelle ein drittes Schaltelement, vorzugsweise eine elektronische Last, auf, mittels welchem der Gleichanteil in seiner Höhe eingestellt wird.

Diese Ausgestaltung hat den Vorteil, dass der Gleichanteil einfacher dem gepulsten Signalteil zu- und / oder weggeschaltet werden kann aufgrund dessen, dass der Gleichanteil mittels eines separaten Schaltelements eingestellt wird. Weiterhin weist diese Ausgestaltung Vorteile hinsichtlich der Kosten und des schaltungstechnischen Aufwandes auf. Es können herkömmliche und vor allem kostengünstige Bauteile zu Realisierung herangezogen werden.

Gemäß einer bevorzugten Weiterbildung wird die Beleuchtungseinheit, insbesondere die LEDs, für die gesamte Dauer der Ansteuerung mit dem Gleichanteil beaufschlagt. Für die gesamte Dauer der Ansteuerung wird das Halbleitermaterial des Strahlungsdetektors somit mit einem dauerhaften Licht beleuchtet. Der Gleichanteil wird dem gepulsten Signalteil zugeschaltet um das Halbleitermaterial zu konditionieren. Hierdurch ist eine genauere Ermittlung der Polarisation ermöglicht.

Vorzugsweise weisen die eingestellten Einzelpulse eine Flankensteilheit mit einem Wert im Bereich von 10ns bis 15ns, insbesondere von maximal 20ns auf.

Gemäß einer zweckdienlichen Ausgestaltung werden die Einzelpulse derart eingestellt, dass sie eine Pulsbreite in einem Bereich von 80ns bis 120ns und vorzugsweise in einem Bereich von 90ns und 110ns aufweisen.

Die beschriebene Flankensteilheit sowie die beschriebene Pulsbreite haben sich als geeignet erwiesen, um innerhalb des Halbleitermaterials des Strahlungsdetektors die Elektronen-Transporte zu generieren.

Die Aufgabe wird weiterhin gelöst durch einen Strahlungsdetektor mit den Merkmalen des Anspruchs 9. Der eingangs beschriebene Strahlungsdetektor, insbesondere Röntgendetektor, weist eine Ansteuervorrichtung für eine Beleuchtungseinheit, insbesondere für eine LED-Einheit auf. Die Ansteuervorrichtung weist eine Pulsstromquelle und eine Gleichstromquelle (DC-Stromquelle) auf.

Durch die Ausgestaltung der Ansteuervorrichtung mit der Aufteilung in die Pulsstromquelle und die DC-Stromquelle ist ein einfacher und kostengünstiger Aufbau realisiert.

Weiterhin sind die Pulsstromquelle und die DC-Stromquelle parallel zueinander geschaltet. Der Parallelschaltung liegt die Überlegung zugrunde, dass auf diese Weise einer Überlagerung der beiden Signale Rechnung getragen wird. Zweckdienlicherweise weist die Pulsstromquelle ein erstes Schaltelement und ein sich an das erste Schaltelement seriell anschließendes zweites Schaltelement auf. Das erste Schaltelement dient zur Einstellung der Amplitude (Pulshöhe) des gepulsten Signalteils und das zweite Schaltelement dient zur Einstellung des Pulsmusters des gepulsten Signalteils. Hierfür weisen das erste Schaltelement vorzugsweise eine elektronische Last - auch Stromsenke genannt - und das zweite Schaltelement vorzugsweise einen Schalter auf.

Elektronische Lasten sind elektrotechnische Bauteile oder Baugruppen aus Bauteilen, welche üblicherweise als Ersatz für einen Lastwiderstand in einer Schaltung angeordnet sind. Der Unterschied zwischen einer elektronischen Last und beispielsweise einem (konventionellen) ohmschen Widerstand ist in der Einstellbarkeit der elektronischen Last zu sehen. Mit anderen Worten: Während über einen an eine Spannungsquelle angeschlossenen ohmschen Widerstand ein elektrischer Strom fließt, welcher einen konstanten Wert aufweist, ist der elektrische Strom, der über eine elektronische Last fließt innerhalb eines definierten Wertebereiches einstellbar, beispielsweise mittels einer elektronischen Regelung.

Elektronische Lasten weisen zudem vorzugsweise elektronische Schalter, insbesondere Transistoren, zur Einstellbarkeit des elektrischen Stromes auf.

Sowohl die elektronische Last des ersten Schaltelements als auch der Schalter des zweiten Schaltelements weist bevorzugt einen elektronischen Schalter, insbesondere einen Transistor auf.

Ein Unterschied zwischen den in den Schaltelementen angeordneten elektronischen Schaltern besteht in der Schaltzeit, welche die elektronischen Schalter aufweisen. Die Schaltzeit ist unmittelbar mit einer Transit-Frequenz (transistion frequency) korreliert, welche die Schaltfrequenz eines Transistor beschreibt. Zur Einstellung der Pulshöhe des gepulsten Signalteils weist das erste Schaltelement einen elektronischen Schalter, insbesondere einen Transistor auf, welcher vorzugsweise eine Transitfrequenz mit einem Wert im Bereich von 100MHz bis 200MHz aufweist. Dieser, für elektronische Schalter, langsamen Schaltfrequenz liegt die Überlegung zugrunde, dass für die Einstellung der Pulshöhe des gepulsten Signalteils, insbesondere unter Berücksichtigung des Aspekts, dass die Pulshöhe bevorzugt nach Art einer Treppenform gesteigert wird, eine Schaltfrequenz in dem angegebenen Wertebereich den Anforderungen gerecht wird.

Das zweite Schaltelement weist zweckdienlicherweise einen elektronischen Schalter, insbesondere einen Transistor auf, welcher eine Transitfrequenz mit einem Wert im Bereich von 500MHz bis 2GHz, insbesondere mit einem Wert im Bereich zwischen 1GHz und 1,5GHZ aufweist. Bevorzugt weist das zweite Schaltelement allgemein beispielsweise eine zumindest um den Faktor 5, bevorzugt um den Faktor 10 höhere Schaltfrequenz als eine geforderte Schaltfrequenz auf, welche sich aus einer geforderten Flankensteilheit errechnet. Somit ist das zweite Schaltelement zur Einstellung des Pulsmusters, welches vorzugsweise eine Flankensteilheit mit einem Wert im Bereich zwischen 10ns und 20ns aufweist, ausgelegt.

Der Vorteil ist darin zu sehen, dass aufgrund der aufgeteilten Erzeugung des gepulsten Signalteils eine Verwendung von kostengünstigen Bauteilen, insbesondere bei den Bauteilen des ersten Schaltelements, ermöglicht ist, wodurch die Ansteuervorrichtung trotzdem die gleichen Anforderungen erfüllt wie die herkömmlichen teuren Ansteuervorrichtungen.

Bevorzugt weist die DC-Stromquelle ein drittes Schaltelement zur Einstellung des Gleichanteils auf in dem vorzugsweise ein elektronischer Schalter, insbesondere ein Transistor angeordnet ist, welcher eine Transitfrequenz mit einem Wert im Bereich von 100MHz bis 200MHz aufweist. Aufgrund der dauerhaften Beleuchtung des Halbleitermaterials ist der Vorteil dieser Ausgestaltung entgegen einer schnellen Schaltzeit in der kostengünstigen Fertigung des Schaltelementes aufgrund einfacherer Bauteile zu sehen. Weiterhin weisen bevorzugt die elektronischen Schalter des ersten Schaltelements und des dritten Schaltelement die gleichen Bauteile auf.

Die im Hinblick auf das Verfahren aufgeführten Vorteile und bevorzugten Ausgestaltungen sind sinngemäß auf die Vorrichtung zu übertragen und umgekehrt.

Ausführungsbeispiele der Erfindung werden nachfolgend anhand der Figuren näher erläutert. Diese zeigen teilweise in stark vereinfachten Darstellungen:
- FIG 1: einen Schaltplan einer Beleuchtungseinheit und einer an die Beleuchtungseinheit angeschlossenen Ansteuervorrichtung sowie
- FIG 2: eine schamtische, ausschnittsweise Darstellung eines Ansteuersignals.

In den Figuren sind gleichwirkende Teile mit den gleichen Bezugszeichen dargestellt.

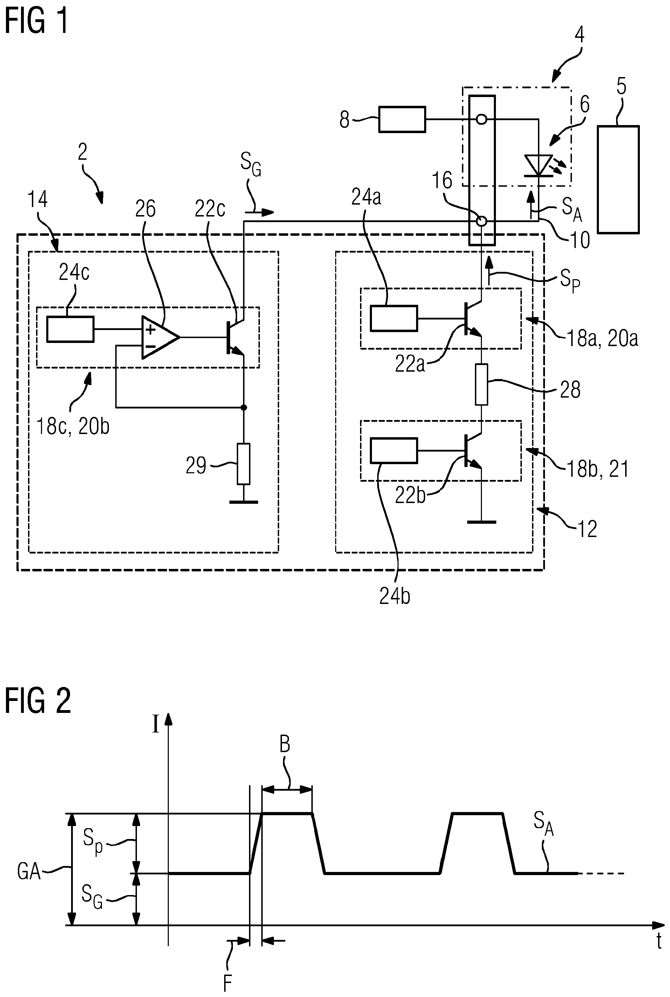

In FIG 1 ist ein vereinfachter Schaltplan einer Ausführungsvariante einer Ansteuervorrichtung 2 für eine Beleuchtungseinheit 4, insbesondere eine LED-Einheit 6 dargestellt. Die Beleuchtungseinheit 4 dient zum Beleuchten eines Strahlungsdetektors 5. Bei dem Strahlungsdetektor 5 handelt es sich um einen eingangs beschriebenen Röntgenstrahlungsdetektor.

Unter LED-Einheit 6 wird vorliegend eine Anordnung mehrerer einzelner LEDs nach Art eines Arrays verstanden. Weiterhin weist die Beleuchtungseinheit 4 eine Versorgungseinheit 8 zur Versorgung der LED-Einheit 6 und der Ansteuervorrichtung 2 mit einer Versorgungsspannung auf. Die Ansteuervorrichtung 2 und die Beleuchtungseinheit 4 sind mittels einer Leitung 10 elektrisch miteinander verbunden. Die LED-Einheit 6 weist herkömmliche LEDs oder alternativ Infrarot-LEDs auf.

Die Ansteuervorrichtung 2 sowie die Beleuchtungseinheit 4 sind zu einem Beleuchten eines Halbleitermaterials eines Strahlungsdetektors, insbesondere Röntgenstrahlungsdetektors ausgebildet. Mit Hilfe der Beleuchtung ist eine Ermittlung eines Polarisationszustandes des Strahlungsdetektors ermöglicht.

Zur Beleuchtung des Halbleitermaterials ist die LED-Einheit 6 mit einem Ansteuersignal S_{A} beaufschlagt, welches mittels der Ansteuervorrichtung 2 erzeugt wird.

Hierzu weist die Ansteuervorrichtung 2 im Ausführungsbeispiel eine Pulsstromquelle 12 und eine Gleichstromquelle (DC-Stromquelle) 14 auf.

Das Ansteuersignal S_{A} weist somit zwei Signalteile, einem gepulsten Signalteil S_{P} und einem Gleichanteil S_{G} auf, welche sich in einem Knotenpunkt 16 der Ansteuervorrichtung zur Erzeugung des eigentlichen Ansteuersignals S_{A} addieren.

Die Pulsstromquelle 12 ist zur Erzeugung des gepulsten Signalteils S_{P} ausgebildet. Hierzu weist die Pulsstromquelle 12 ein erstes Schaltelement 18a und ein sich an das erste Schaltelement 18a seriell anschließendes zweites Schaltelement 18b auf.

Das erste Schaltelement 18a weist zur Einstellung einer Pulshöhe des gepulsten Signals S_{G} eine erste elektronische Last 20a auf. Die erste elektronische Last 20a weist einen ersten elektronischen Schalter 22a, im Ausführungsbeispiel einen Transistor, beispielsweise einen Transistor vom Typ BC817 auf. Weiterhin weist die erste elektronische Last 20a zur Steuerung des ersten elektronischen Schalters 22a eine erste Steuereinheit 24a, im Ausführungsbeispiel einen Digital-Analog-Konverter auf.

Das zweite Schaltelement 18b weist zur Einstellung eines Pulsmusters des gepulsten Signals S_{G} einen Schalter 21 auf. Der Schalter 21 weist insbesondere einen zweiten elektronischen Schalter 22b, im Ausführungsbeispiel einen Transistor, beispielsweise einen Transistor vom Typ BFU590G auf. Weiterhin weist das zweite Schaltelement 18b zur Regelung und Einstellung des Pulsmusters eine an den zweiten elektronischen Schalter 22b angeschlossene zweite Steuereinheit, im Ausführungsbeispiel ein Field-Programmable-Gate-Array (FPGA) auf.

Unterschiede in den beiden Schaltelementen 18a, b sind im Wesentlichen in verschiedenen Anforderungen hinsichtlich der Schaltzeiten, insbesondere der Transitfrequenzen der beiden elektronischen Schalter 22a, b zu sehen.

Eine Anforderung an die Schaltzeit des zweiten elektronischen Schalters 22b ergibt sich aus einer Anforderung einer maximal zulässigen Flankensteilheit eines einzustellenden Pulsmusters. Im Ausführungsbeispiel weist der gepulste Signalteil ein Pulsmuster nach Art eines Rechtecksignals auf.

Der zweite elektronische Schalter 22b weist bevorzugt eine Transitfrequenz mit einem Wert im Bereich von 500MHz bis 2GHz, insbesondere mit einem Wert im Bereich zwischen 1GHz und 1,5GHz. Mit Transitfrequenzen mit einem Wert innerhalb der genannten Bereiche können Flankensteilheiten mit einem Wert im Bereich von 10ns bis 20ns erreicht werden.

Auch höhere Transitfrequenzen sind denkbar, beispielsweise weist der zweite elektronische Schalter 22b im Ausführungsbeispiel einen Transistor vom Typ BFU590G auf, welcher eine Transitfrequenz von 8,5GHz aufweist.

Gemäß der im Ausführungsbeispiel bevorzugten Ausgestaltung der Steigerung der Pulshöhe (Amplitude) der Einzelpulse des gepulsten Signalteils S_{G} erfolgt die Steigerung nach Art einer Treppenform. Somit ergibt sich eine Schaltzeit, respektive eine Transitfrequenz des ersten elektronischen Schalters 22a, welche einen geringeren Wert als den Wert der Transit-frequenz des zweiten elektronischen Schalters 22b aufweist. Diese Überlegung liegt zugrunde, dass zum Erreichen der Treppenform lediglich nach einer Pulsfolge, die einem Vielfachen einer Einzelpulsdauer entspricht, die Pulshöhe (Amplitude) der Einzelpulse erhöht wird. Im Ausführungsbeispiel weist die Transitfrequenz des ersten elektronischen Schalters 22a vom Typ BC817 einen Wert von 170MHz auf.

Typischerweise weist eine Transitfrequenz eines elektronischen Schalters einen um den Faktor 10 höheren Wert auf, als ein Wert einer in eine Frequenz umgerechnete gewünschte Flankensteilheit, welche mittels des elektronischen Schalters realisiert werden soll. Beispielsweise weist ein elektronischer Schalter zur Realisierung einer Flankensteilheit von 10ns, was einer Frequenz von 100MHz entspricht, eine Transitfrequenz von 1GHz auf.

Die DC-Stromquelle 14 ist zur Erzeugung des Gleichanteils S_{G} ausgebildet. Hierzu weist die DC-Stromquelle 14 ein drittes Schaltelement 18c auf. Das dritte Schaltelement 18c weist zur Einstellung des Gleichanteils S_{G} einerseits eine dritte Steuereinheit 24c, beispielsweise einen Digital-Analog-Wandler und andererseits eine Stromsenke20b auf. Die Stromsenke 20b weist einen dritten elektronischen Schalter 22c, beispielsweise einen Transistor vom Typ BC817 auf. Weiterhin weist das dritte Schaltelement 18c einen Operationsverstärker 26 zur analogen Regelung des Gleichanteils S_{G} auf. Mittels der dritten Steuereinheit 24c erfolgt die Einstellung, beispielsweise einer Amplitude des Gleichanteils S_{G}, welcher mittels des dritten elektronischen Schalters 22c zuschaltbar dem gepulsten Signalteil S_{G} zugeführt wird.

Typischerweise und bevorzugt ist die LED-Einheit 6 im Betrieb dauerhaft mit dem Gleichanteil S_{G} beaufschlagt. Somit ergeben sich für den dritten elektronischen Schalter 22c Anforderungen hinsichtlich einer Schaltzeit und / oder einer Transit-frequenz, welche bevorzugt mittels eines herkömmlichen Transistors, beispielsweise eines Bipolartransistors vom Typ BC817 erfüllt sind.

Ergänzend weist die gepulste Stromquelle ein Strombegrenzerelement 28, beispielsweise einen Emitterwiderstand, zur Kompensation einer Temperaturdrift des ersten elektronischen Schalters 22a auf. Unter Temperaturdrift wird allgemein eine temperaturabhängige Stromverstärkung des Transistors verstanden.

Weiterhin weist die DC-Stromquelle ein Strommesselement 29 beispielsweise einen Shunt-Widerstand zu einer Erfassung einer Höhe des Gleichanteils S_{G} auf.

Zum besseren Verständnis wird im Folgenden noch einmal kurz auf das bereits in der DE 10 2015 201 494 A1 beschriebene Verfahren eingegangen:

Die LEDs werden mit dem gepulsten Signalteil beaufschlagt. Aufgrund der Zunahme der Amplitude der Einzelpulse und somit einer Zunahme des LED-Stromes, welcher durch die LEDs fließt, weist das Licht der LEDs eine zunehmende Intensität auf. Durch die zunehmende Intensität des Lichtes der LEDs wird eine zunehmende Anzahl an Ladungsträger-Paaren innerhalb eines Halbleitermaterials des Strahlungsdetektors 5 generiert, was zu einem höheren Ladungspuls führt. Nach jeder Zunahme der Amplitude der Einzelpulse und somit nach jeder Zunahme des Ladungspulses wird dieser mit einer voreingestellten Schwellenspannung einer Signalerfassungseinheit verglichen. Weist der Ladungspuls beispielsweise einen kleineren Wert als die Schwellenspannung auf, wird beispielsweise ein Zählereignis innerhalb der Signalerfassungseinheit erfasst und die Amplitude der Einzelpulse mittels der Ansteuervorrichtung 2 erhöht. Nach der Erhöhung erfolgt erneut ein Vergleich des Ladungspulses mit der Schwellenspannung. Überschreitet beispielsweise der Wert des Ladungspulses den Wert der Schwellenspannung, so wird das Zählereignis als ein Wert zur Beurteilung der Polarisation des Strahlungsdetektors genutzt. Unter Zählereignis wird verstanden, wie oft eine Zunahme der Einzelpulse erfolgte, bis der Wert des Ladungspulses den Wert der Schwellenspannung überschritten hat.

Aufgrund der zeitlichen Veränderung der Polarisation und aufgrund der Beeinflussung von Strahlung auf den Polarisationszustand des Strahlungsdetektors wird das Verfahren zur Bestimmung der Polarisation vorzugsweise einmal vor einer Behandlung eines Patienten und einmal nach einer Behandlung des Patienten durchgeführt. Bei beiden Messungen wird jeweils ein gleicher Wert für die Schwellenspannung eingestellt. Beispielsweise kann bei der Messung nach der Behandlung und somit nachdem der Strahlungsdetektor Röntgenstrahlung ausgesetzt war ein schnelleres oder langsameres Überschreiten der Schwellenspannung festgestellt werden. Die Differenz zwischen einer Anzahl an Einzelimpulszunahmen bis zur Überschreitung der Schwellenspannung bei der Messung vor der Behandlung und einer Anzahl an Einzelimpulszunahmen bis zur Überschreitung der Schwellenspannung bei der Messung nach der Behandlung wird als Signaldrift gedeutet und wird anschließend beispielsweise mittels Kalibierverfahren reduziert.

FIG 2 zeigt eine schematische Ausschnittsdarstellung eines Ansteuersignals S_{A}. Hierbei ist der Wert I des Ansteuersignals auf einer senkrechten Stromachse in Abhängigkeit der auf einer waagerechten Zeitachse aufgetragenen Zeit t aufgetragen. Im Ausführungsbeispiel weist das Ansteuersignal S_{A} einen gepulsten Signalteil S_{P} nach Art eines Rechtecksignals auf. Zudem ist der gepulste Signalteil S_{P} mit einem Gleichanteil S_{G} überlagert. Die Überlagerung des gepulsten Signalteils S_{P} "verschiebt" den gepulsten Signalteil S_{P} in Richtung der Stromachse um den Wert des Gleichanteils S_{G}. Das Ansteuersignal S_{A} weist somit eine Gesamtamplitude GA auf. Die Gesamtamplitude GA wird aus der Summe des Wertes des Gleichanteils S_{G} und der Amplitude (Pulshöhe) des gepulsten Signalteils S_{P} gebildet.

Im Ausführungsbeispiel weist der gepulste Signalteil S_{P} eine Amplitude A, eine Flankensteilheit F sowie eine Pulsbreite B auf. Die Flankensteilheit F definiert die Zeit, in der der gepulste Signalteil S_{P} von einem Ausgangswert (im Ausführungsbeispiel ist der Ausgangswert der Wert des Gleichanteils S_{G}) auf den vollen Wert seiner Amplitude A ansteigt. Im Ausführungsbeispiel weist die Flankensteilheit F insbesondere einen maximalen Wert von 20ns auf. Die Pulsbreite B definiert eine Zeit, in der das Ansteuersignal S_{A}, respektive der gepulste Signalteil S_{P} seine Amplitude aufweist, bevor der gepulste Signalteil S_{P} zur Ausbildung des Pulsmusters auf seinen Ausgangswert abfällt. Im Ausführungsbeispiel weist die Pulsbreite Wert im Bereich zwischen 100ns und 120ns auf.

## Patentansprüche

1. Verfahren zum Betrieb eines direkt-konvertierenden Strahlungsdetektors (5), der mittels einer Beleuchtungseinheit (4), insbesondere einer LED-Einheit (6), zur Ermittlung einer Polarisation mit Licht beleuchtet wird, wobei die Beleuchtungseinheit (4) mittels einer Ansteuervorrichtung (2) angesteuert wird,
**dadurch gekennzeichnet, dass** die Ansteuervorrichtung (2) eine Gleichstromquelle (14) und eine Pulsstromquelle (12) aufweist, wobei die Beleuchtungseinheit (6) von einem Ansteuersignal (S_{A}) angesteuert wird und das Ansteuersignal (S_{A}) einen gepulsten Signalteil (S_{P}), welcher von der Pulsstromquelle (12) erzeugt wird, und einen über den gepulsten Signalteil (S_{P}) gelagerten Gleichanteil (S_{G}), welcher von der Gleichstromquelle (14) erzeugt wird, aufweist.

2. Verfahren nach dem vorhergehenden Anspruch,
**dadurch gekennzeichnet, dass** der gepulste Signalanteil (S_{P}) eine Amplitude (A) und ein Pulsmuster und die Pulsstromquelle (12) ein erstes Schaltelement (18a) und ein sich an das erste Schaltelement (18a) anschließendes zweites Schaltelement (18b) aufweisen, wobei die Amplitude (A) mittels des ersten Schaltelements (18a) und das Pulsmuster mittels des zweiten Schaltelements (18b) eingestellt wird.

3. Verfahren nach dem vorhergehenden Anspruch,
**dadurch gekennzeichnet, dass** der gepulste Signalteil (S_{P}) eine Folge von Einzelpulsen aufweist, wobei die Amplitude (A) der Einzelimpulse zunimmt.

4. Verfahren nach einem der beiden vorhergehenden Ansprüche, **dadurch gekennzeichnet, dass** auf einer Regelung der Amplitude (A) des gepulsten Signalteils (S_{P}) verzichtet wird.

5. Verfahren nach einem der vorhergehenden Ansprüche,
**dadurch gekennzeichnet, dass** die DC-Stromquelle (14) ein drittes Schaltelement (18c) aufweist, wobei der Gleichanteil (S_{G}) mittels des dritten Schaltelementes (18c) eingestellt wird.

6. Verfahren nach einem der vorhergehenden Ansprüche,
**dadurch gekennzeichnet, dass** die Beleuchtungseinheit (4) für die gesamte Dauer der Ansteuerung mit dem Gleichanteil (S_{G}) beaufschlagt wird.

7. Verfahren nach einem der Ansprüche 3 bis 6,
wobei die Einzelpulse eine Flankensteilheit (F) von maximal 20ns aufweisen.

8. Verfahren nach einem der Ansprüche 3 bis 7,
wobei die Einzelpulse eine Pulsbreite (B) in einem Bereich von 80ns bis 120ns, vorzugsweise in einem Bereich von 90ns bis 110ns aufweisen.

9. Direkt-konvertierender Strahlungsdetektor (5) mit einer Ansteuervorrichtung (2) für eine Beleuchtungseinheit (4), insbesondere für eine LED-Einheit (6), zur Ermittlung einer Polarisation,
**dadurch gekennzeichnet, dass** die Ansteuervorrichtung (2) eine Pulsstromquelle (12) sowie eine Gleichstromquelle (14) aufweist,
wobei die Pulsstromquelle (12) und die Gleichstromquelle (14) zur Ansteuerung der Beleuchtungseinheit (6) ausgebildet sind, derart, dass die Beleuchtungseinheit (6) mit Hilfe eines Ansteuersignals (S_{A}) angesteuert wird, wobei das Ansteuersignal (S_{A}) einen gepulsten Signalanteil (S_{P}), welcher von der Pulsstromquelle (12) erzeugt wird, und eine über den gepulsten Signalteil (S_{P}) lagerbaren Gleichanteil (S_{G}), welcher von der Gleichstromquelle (14) erzeugt wird, aufweist.

10. Strahlungsdetektor (5) nach dem vorhergehenden Anspruch, **dadurch gekennzeichnet, dass** die Pulsstromquelle (12) ein erstes Schaltelement (18a) und ein sich an das erste Schaltelement (18a) anschließendes zweites Schaltelement (18b) aufweist, wobei das erste Schaltelement (18a) zur Einstellung einer Amplitude (A) des gepulsten Signalteils (S_{P}) ausgebildet ist und das zweite Schaltelement (18b) zur Einstellung eines Pulsmusters des gepulsten Signalteils (S_{P}) ausgebildet ist.

11. Strahlungsdetektor (5) nach einem der beiden vorhergehenden Ansprüche,
**dadurch gekennzeichnet, dass** die Transitfrequenz des zweiten Schaltelements (18b) einen, insbesondere um den Faktor 5 größeren Wert als der Wert einer aus einer geforderten Flankensteilheit (F) ermittelten Transitfrequenz aufweist.

12. Strahlungsdetektor (5) nach einem der Ansprüche 9 bis 11, **dadurch gekennzeichnet, dass** die Gleichstromquelle (14) ein drittes Schaltelement (18c) zur Einstellung des Gleichanteils (S_{G}) aufweist.

## Claims

1. Method for operating a direct-converting radiation detector (5), which is illuminated with light by means of an illumination unit (4), in particular an LED unit (6), for determining a polarisation, wherein the illumination unit (4) is controlled by means of a control device (2), **characterised in that** the control device (2) has a DC source (14) and a pulse current source (12), wherein the illumination unit (6) is controlled by a control signal (S_{A}) and the control signal (S_{A}) has a pulsed signal component (S_{P}), which is generated by the pulse current source (12), and a direct component (S_{G}) superimposed over the pulsed signal component (S_{P}), which is generated by the DC source (14).

2. Method according to the preceding claim,
**characterised in that** the pulsed signal component (S_{P}) has an amplitude (A) and a pulse pattern and the pulse current source (12) has a first switching element (18a) and a second switching element (18b) following on from the first switching element (18a), wherein the amplitude (A) is set by means of the first switching element (18a) and the pulse pattern by means of the second switching element (18b).

3. Method according to the preceding claim,
**characterised in that** the pulsed signal component (S_{P}) has a sequence of individual pulses, wherein the amplitude (A) of the individual pulses increases.

4. Method according to one of the two preceding claims,
**characterised in that** control of the amplitude (A) of the pulsed signal component (S_{P}) is dispensed with.

5. Method according to one of the preceding claims, **characterised in that** the DC source (14) has a third switching element (18c), wherein the direct component (S_{G}) is set by means of the third switching element (18c).

6. Method according to one of the preceding claims, **characterised in that** the direct component (S_{G}) acts upon the illumination unit (4) for the entire duration of the control.

7. Method according to one of claims 3 to 6,
wherein the individual pulses have a maximum edge steepness (F) of 20 ns.

8. Method according to one of claims 3 to 7,
wherein the individual pulses have a pulse width (B) in a range between 80 ns and 120 ns, preferably in a range between 90 ns and 110 ns.

9. Direct-converting radiation detector (5) with a control device (2) for an illumination unit (4), in particular for an LED unit (6), for determining a polarisation, **characterised in that** the control device (2) has a pulse current source (12) and a DC source (14),
wherein the pulse current source (12) and the DC source (14) are designed to control the illumination unit (6), such that the illumination unit (6) is controlled with the help of a control signal (S_{A}), wherein the control signal (S_{A}) has a pulsed signal component (S_{P}), which is generated by the pulse current source (12), and a direct component (S_{G}) which can be superimposed over the pulsed signal component (S_{P}) and which is generated by the DC source (14).

10. Radiation detector (5) according to the preceding claim, **characterised in that** the pulse current source (12) has a first switching element (18a) and a second switching element (18b) following on from the first switching element (18a), wherein the first switching element (18a) is designed to set an amplitude (A) of the pulsed signal component (S_{P}) and the second switching element (18b) is designed to set a pulse pattern of the pulsed signal component (S_{P}).

11. Radiation detector (5) according to one of the two preceding claims,
**characterised in that** the transition frequency of the second switching element (18b) has a value greater, in particular by a factor of 5, than the value of a transition frequency determined from a required edge steepness (F).

12. Radiation detector (5) according to one of claims 9 to 11, **characterised in that** the DC source (14) has a third switching element (18c) to set the direct component (S_{G}).

## Revendications

1. Procédé pour faire fonctionner un détecteur (5) de rayonnement à conversion directe, qui, au moyen d'une unité (4) d'éclairage, en particulier d'une unité (6) DEL, est éclairé par de la lumière pour la détermination d'une polarisation, dans lequel on commande l'unité (4) d'éclairage au moyen d'un dispositif (2) de commande, **caractérisé en ce que** le dispositif (2) de commande a une source (14) de courant continu et une source (12) de courant pulsé, dans lequel on commande l'unité (6) d'éclairage par un signal (S_{A}) de commande et le signal (S_{A}) de commande a une partie (S_{P}) de signal pulsée, qui est produite par la source (12) de courant pulsé, et une partie (S_{G}) continue, qui est superposée à la partie (S_{P}) de signal pulsée et qui est produite par la source (14) de courant continu.

2. Procédé suivant la revendication précédente,
**caractérisé en ce que** la partie (S_{P}) de signal pulsée a une amplitude (A) et un modèle d'impulsion et la source (12) de courant pulsé a un premier élément (18a) de coupure et un deuxième élément (18b) de coupure se raccordant au premier élément (18a) de coupure, dans lequel on règle l'amplitude (A) au moyen du premier élément (18a) de coupure et le modèle d'impulsion au moyen du deuxième élément (18b) de coupure.

3. Procédé suivant la revendication précédente,
**caractérisé en ce que** la partie (S_{P}) de signal pulsée a une séquence d'impulsions individuelles, dans lequel l'amplitude (A) de l'impulsion individuelle augmente.

4. Procédé suivant l'une des revendications précédentes, **caractérisé en ce que** l'on renonce à une régulation de l'amplitude (A) de la partie (S_{P}) de signal pulsée.

5. Procédé suivant l'une des revendications précédentes, **caractérisé en ce que** la source (14) de courant continu a un troisième élément (18c) de coupure, dans lequel on règle la partie (S_{G}) continue au moyen du troisième élément (18c) de coupure.

6. Procédé suivant l'une des revendications précédentes, **caractérisé en ce que** l'on alimente en la partie (S_{G}) continue l'unité (4) d'éclairage pendant toute la durée de la commande.

7. Procédé suivant l'une des revendications 3 à 6,
dans lequel les impulsions individuelles ont une raideur (F) de flanc de 20 ns au maximum.

8. Procédé suivant l'une des revendications 3 à 7,
dans lequel les impulsions individuelles ont une largeur (B) d'impulsion dans une plage de 80 ns à 120 ns, de préférence dans une plage de 90 ns à 110 ns.

9. Détecteur (5) de rayonnement à conversion directe, comprenant un dispositif (2) de commande d'une unité (4) d'éclairage, en particulier d'une unité (6) DEL, pour la détermination d'une polarisation,
**caractérisé en ce que** le dispositif (2) de commande a une source (12) de courant pulsé ainsi qu'une source (14) de courant continu,
dans lequel la source (12) de courant pulsé et la source (14) de courant continu sont constituées pour la commande de l'unité (6) d'éclairage,
de manière à ce que l'unité (6) d'éclairage soit commandée à l'aide d'un signal (S_{A}) de commande, dans lequel le signal (S_{A}) de commande a une partie (S_{P}) de signal pulsée, qui est produite par la source (12) de courant pulsé, et une partie (S_{G}) continue, qui peut être superposée à la partie (S_{P}) de signal pulsée et qui est produite dans la source (14) de courant continu.

10. Détecteur (5) de rayonnement suivant la revendication précédente,
**caractérisé en ce que** la source (12) de courant pulsé a un premier élément (18a) de coupure et un deuxième élément (18b) de coupure se raccordant au premier élément (18a) de coupure, dans lequel le premier élément (18a) de coupure est constitué pour le réglage d'une amplitude (A) de la partie (S_{P}) de signal pulsée et le deuxième élément (18b) de coupure pour le réglage d'un modèle d'impulsion de la partie (S_{P}) de signal pulsée.

11. Détecteur (5) de rayonnement suivant l'une des deux revendications précédentes,
**caractérisé en ce que** la fréquence de transit du deuxième élément (18b) de coupure a une valeur, en particulier plus grande du facteur de 5, que la valeur d'une fréquence de transit déterminée à partir d'une raideur (F) de flanc exigée.

12. Détecteur (5) de rayonnement suivant l'une des revendications 9 à 11,
**caractérisé en ce que** la source (14) de courant continu a un troisième élément (18c) de coupure pour le réglage de la partie (S_{G}) continue.
